# EUROPEAN PATENT SPECIFICATION

(11) **EP 0 366 029 B1**
(45) Date of publication and mention of the grant of the patent: **07.09.1994**
(21) Application number: 89119571.1
(22) Date of filing: 21.10.1989
(51) Int. Cl.: A61L 27/00, A61L 25/00

(54) **Bone repairing material and artificial bone fixing agent**
Knochenersatzmaterial und Knochenzement
Matériau pour la réparation des os et ciment osseux

(30) Priority: 25.10.1988 JP 268874/88
(43) Date of publication of application: 02.05.1990
(73) Proprietor: Yamamuro, Takao, Mukou Kyoto 617 (JP)
(72) Inventor: Yamamuro, Takao, Mukou Kyoto 617 (JP); Nakamura, Takashi, Ukyo-ku Kyoto 615 (JP); Kotani, Seiya, Sakyo-ku Kyoto 606 (JP)
(74) Representative: von Kreisler, Alek, Dipl.-Chem.

(56) References cited:
- EP-A- 0 082 621
- EP-A- 0 182 483
- FR-A- 2 564 732
- GB-A- 2 176 192
- US-A- 4 563 489
- US-A- 4 596 574
- US-A- 4 627 982

## Description

### BACKGROUND OF THE INVENTION

### 1. Field of the Invention

The present invention relates to a bone repairing material. More particularly, the invention relates to a bone repairing material comprising an AW-glass ceramic and an osteogenesis promoting substance in combination therewith.

### 2. Description of the Prior Art

Conventionally, bone grafting in the living body is performed generally by autografting with a bone of the patient himself. However, the bone thus available for autografting is limited in quantity, while the patient must undergo surgery twice for the removal of the bone and the transplantation thereof and therefore suffers pain.

To overcome the drawback of autografting, it has been attempted to prepare a demineralized powder of organic matrix from the bone of a mammal (hereinafter referred to as a "demineralized bone component") and transplant the demineralized bone component in the living body to form an autogenous bone (Glowacki et al., Lancet, May 12, 959, 1981). The demineralized bone component contains several kinds of local bone repair regulating factors. When the component is transplanted in the living body, granulations are formed around the particles of the demineralized bone component through a vital reaction. Subsequently, mesenchymal cells in the living body are drawn to the surfaces of the particles of the component by a chemotactic factor present in the bone component and are differentiated into cartilage cells by a differentiation promoting factor present in the component. When blood capillaries are further formed in the granulation tissue, the cartilage cells die. The mesenchymal cells are differentiated into osteoblasts by a factor released from the cartilage cells and a factor released from the component. The osteoblasts calcify the extracellular matrix and secrete collagen into the interstices between the particles of the bone component, further calcifying the secreted collagen, with the result that a normal bone is formed in the interior of the granulation tissue. The bone thus formed undergoes a metabolic turnover as a normal bone.

The drawback involved in the use of an autogenous bone for supplementing the site of fracture can be remedied by using the demineralized bone component of a mammal, whereas the component itself has no strength, such that when the bone component is used singly, it requires a long period of 1.5 to 2 years for the bone formed to exhibit strength to withstand external forces. Further in the case where the component is prepared from a bone from a species different from the recipient, a satisfactory bone will not always be formed owing to the inherent antigenicity, so that antigenic components are removed by rough extraction (Sampath et al., Proc. Nalt. Acad. Sci., U.S.A, 84, 7109(1987)).

Further, GB 2 176 192 discloses a filler consisting of calcium phosphate compound and a bone morphogenetic protein;
FR 2 564 732 discloses a biomaterial for osteogenesis substance consisting of a first carrier (including collagen) and a second carrier. The second carrier includes ceramic;
US 4 596 574 discloses an osteogenesis morphogenetic protein (BMP) delivery system comprising a porous ceramic including a substantially pure BMP and being used in the bone implantation. It also describes that porous ceramic can be of glass-like, and concretely include tricalcium phosphate (TCP), β-tricalcium phosphate (BTCP);
US 4 563 489 discloses a BMP delivery system comprising a composition constituting a mixture of polymer and osteogenesis morphogenetic protein (BMP), to be utilized in bone implant. Polymers include organic polymers, such as poly(hydroxy organic carboxylic acids);
EP 0 082 621 discloses an implantable bone prosthesis comprising a porous casing containing small pieces of demineralized bone, dentin or a mixture thereof. It also describes that the porous casing is formed of natural or synthetic fibrous or microporous polymers;
US 4 627 982 discloses a method for obtaining a bone-inducing factor partially purified from demineralized bones, the factor thus obtained and an implantation material including this factor. It describes a composition comprising a solid carrier like BCP; and
EP 0 182 483 discloses an implantable material for inductive bone repair comprising both a chemically defined hypoimmunogenic supporting matrix for bone growth and an effective amount of purified osteoinductive factor. The supporting matrix contains at least 5 % of non-fibrillar collagen. It also describes that the matrix can contain such material as fibrillar collagen, hydroxy apatite, tricalcium phosphate (ceramic) and a mixture thereof.

On the other hand, artificial bones of metal, ceramic or the like have been developed for substituting for bone support mechanisms, but these materials have no bone-bonding ability.

The main object of the present invention is to provide a bone repairing material and an artificial bone fixing agent for inducing healing of the defective portion of a natural bone and firmly fixing an artificial bone to the natural bone.

### SUMMARY OF THE INVENTION

The present invention provides a bone repairing material which comprises an AW-glass ceramic and an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction, and applied to and/or incorporated in the AW-glass ceramic.

The invention further relates to a process for preparing a bone repairing material comprising applying to and/or incorporating in an AW-glass ceramic an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction; and to the use of an AW-glass and an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction, for preparing a bone repairing material for inducing bone formation at a predetermined site in a mammal comprising implanting said AW-glass ceramic at the site, wherein said ceramic has said osteogenesis promoting substance applied to and/or incorporated in.

The present invention is based on the fact that when an osteogenesis promoting substance having a bone-bonding ability but no supporting function is used in combination with an artificial bone having a supporting function but no bone-bonding ability to fill up a natural bone deficient portion of the living body, an autogenous bone is formed in the filled-up portion around the artificial bone, whereby the artificial bone is made to have the same function and form as the corresponding bone of the living body.

The bone repairing material of the present invention is advantageously usable in surgery or orthopedics for limb salvage and for the treatment of bones, e.g. , comminuted fracture, prolonged fracture,scoliosis, joint prosthesis, and deformities or abnormalities of bones, and in dentistry or oral surgery for forming artificial fangs, alveolar bones and the like for the treatment of alveolar pyorrhea.

According to the present invention, an artificial bone is transplanted in a bone deficient site of the host in combination with the osteogenesis promoting substance, whereby the deficient portion is given sufficient strength by the artificial bone, which in turn becomes firmly bonded to the living tissue in the site of transplantation owing to the bone-bonding ability of the osteogenesis promoting substance.

The bone repairing material of the present invention is thus adapted to form a bonding between the natural bone and the artificial bone with as high a strength as is naturally available and exhibit a high support strength.

### DESCRIPTION OF THE PREFERRED EMBODIMENTS

The term "osteogenesis promoting substance" derived from the bone of an animal for use in the invention refers to a powder of organic matrix or ground substance (hereinafter referred to as "demineralized bone powder") prepared from bones of vertebrates which are readily available, e.g., bones of mammal such as bovines, swines and rabbits, by removing inorganic components (ash) thereof, and to a substance containing a bone repair regulating factor and obtained by purifying the powder. More specifically, the demineralized bone powder means the one disclosed, for example, in the following literature:
Glowacki et al., Lancet May 12, 959, 1981,
Glowacki et al., Clinic in Plastic Surgery, 12, 233(1985),
Sampath et al., Proc. Nalt. Acad. Sci., U.S.A., 84, 7109(1987), etc.
The substance containing a bone repair regulating factor means the one disclosed, for example, in the following literature:
Urist et al., Science, 220, 680(1983),
Sampath et al., Proc. Nalt. Acad. Sci., U.S.A., 84, 7109(1987),
Farley et al., Biochemistry, 21, 3502(1982), etc.
To avoid the rejection due to an antigen-antibody reaction, the mammal to be selected for providing the osteogenesis promoting substance is preferably of the same species as the recipient. However, when the host is a human, the shaft of the long bone of a rabbit or bovine, especially neonatal calf, is desirable.

The demineralized bone powder is prepared, for example, by the following method. The bone to be used as the material is cut and crushed, and the soft tissue is removed from the bone fragments, which are then washed with water, thereafter repeatedly washed with organic solvents such as ethanol and ether, dewatered and de-fatted. The resulting bone fragments are pulverized to a suitable particle size. The particulate bone is then demineralized with hydrochloric acid, ethylenediaminetetraacetic acid or the like, washed with water, then repeatedly washed with an organic solvent and thereafter dried, giving the desired bone powder. The bone of a neonatal calf, when to be used, is collected immediately after the animal is killed, and is frozen for preservation. The bone shaft is cut off and roughly crushed. After removing the soft tissue from the bone fragments, the fragments are washed with water, then repeatedly washed with ethanol and ether, dewatered and de-fatted. With cooling, the resulting bone fragments are promptly pulverized to a suitable particle size. The particulate bone is then demineralized with hydrochloric acid, then washed with water, further washed repeatedly with ethanol and ether, and dried. The powder thus obtained is preferably about 75 to about 450 micrometers in particle size.

The substance containing bone bonding regulating factors is prepared, for example, by subjecting the demineralized bone powder to extraction with an aqueous solution of guanidine hydrochloric acid of a suitable concentration, subjecting the extract to gel filtration using one or two different kinds of filters to obtain a fraction having osteogenetic activity, and lyophilizing the fraction.

The artifical bones to be used in the present invention are those made of known orthopedic materials for use in the living body, such as 1) metal materials, 2) ceramic materials, 3) high polymer materials,and 4) protein materials or composite materials of such materials. However, preferable to use are generally those made of the materials 1) to 3).

Examples of useful metal materials arc titanium, stainless steel and the like. Examples of suitable ceramic materials include bio-inert ceramics such as alumina ceramic, single-crystal alumina ceramic and zirconia ceramic, and bio-active ceramics such as bio-glass (Hench et al., Biomed. Master. Symp., 2, 117(1972)), hydroxyapatite (Aoki et al., Ceramics, 10, 469(1975)) and AW-glass (Bull. Inst. Chem. Res. Kyoto Uni., 60, 260(1982)). Examples of suitable high polymer materials are polymethyl methacrylate (PMMA), high-density polyethylene (HDP), silicone rubber and the like. Examples of protein materials are collagen, fibrin and the like. Such a material is used as shaped in conformity with the shape of the contemplated bone deficient portion to be repaired.

According to the present invention, the osteogenesis promoting substance is applied to or incorporated into the artificial bone. The substance is thus applied or incorporated so that when the artificial bone is filled (transplanted) in the contemplated bone deficient portion, the bone bonding regulating factors contained in the substance will be released in situ into the living tissue of the defective portion. For exmaple, it is desired that the artificial bone has a surface property or surface structure suited to the application or incorporation of the substance. For example, the bone is made porous over the surface. Porous surfaces can be formed by a known method, for example, by bonding together granules of a particular material in the form of two layers with interstices provided between the granules, or by binding continuous metal fibers together in two layers as arranged randomly.

The osteogenesis promoting substance can be applied to or incorporated into such an artificial bone usually by dispersing the substance in a suitable dispersant, binder, diluent or the like (such as collagen, physiological saline, citric acid solution, acetic acid solution, hydroxyapatite, fibrin or mixture thereof), coating or impregnating the bone with the dispersion, and drying the bone. The dispersion is prepared with a concentration sufficient to supply to the artificial bone an effective amount of the osterogenesis promoting substance serving as the active component.

It is desired that the substance be applied or incorporated into the artificial bone at the portion thereof to be firmly bonded to the living tissue of the bone deficient site. The portion is, for example, such that it is in contact with the natural bone of the host when the artificial bone is transplanted. Furthermore, the osteogenesis promoting substance is applied or incorporated into the artificial bone in an amount effective to fully firmlybond the artificial bone to the bone deficient portion of the host. The effective amount is, for example, 0.1 to 1.5 g/cm², preferably about 0.3 to about 0.5 g/cm², based on the area of the portion of the artificial bone to which the substance is to be applied or incorporated.

The bone fixing agent of the present invention is prepared by incorporating the osteogenesis promoting substance as its active component into a dispersant, binder or diluent which is physiologically acceptable. The agent can be thus prepared by a known method. Other components (such as calcium) effective for osteogenesis may be added to the bone fixing agent. Instead of being applied to or incorporated into artificial bones, the bone fixing agent of the invention can be used as filled in a clearance between the bone deficient portion of the host and the artificial bone transplanted therein. In this case, the agent is used in such an effective amount as mentioned above, calculated as the substance.

### EXAMPLES

The present invention will be described in greater detail with reference to the following examples, which nevertheless in no way limit the invention.

### Example 1

Glass ceramic and decalcified bone powder were embedded in tibiae of rabbits and checked for the bone formed around the ceramic.

The glass ceramic used was A-W glass ceramic prepared by the method of Kokubo et al. (Kokubo, T. et al., Bull. Inst. Chem. Res. Kyoto Univ., 60:260, 1982). The ceramic was prepared by a method involving a temperature gradient and was composed of 4.6% of magnesium oxide (MgO), 44.9% of calcium oxide, 34.2% of silicon dioxide (SiO₂), 16.3% of phosphorus pentoxide (P₂O₅) and 0.5% of calcium fluoride (CaF). The ceramic was pulverized, molded into plates, 15 mm in length, 10 mm in width and 2 mm in thickness, and fired again.

The demineralized bone powder was prepared by the method of Glowacki et al. (Glowacki, J. et al., Clinics in Plastic Surgery, 12, 233, 1985). Stated more specifically, the shaft of the long bone of a rabbit was cut off and roughly crushed, and the soft tissue and the bone marrow were removed from the bone fragments, which were then repeatedly washed with cooled deionized water and further repeatedly washed with ethanol and diethyl ether. The bone fragments were then cooled, pulverized by an impact mill and thereafter screened to obtain a bone powder 75 to 450 micrometers in particle size. The bone powder was immersed in 0.5M hydrochloric acid for 3 hours for demineralization, then repeatedly washed with deionized water, ethanol and diethyl ether, and lyophilized to obtain demineralized bone powder.

Bone grafting was done in the following manner. Mature male white rabbits weighing about 3 kg were intravenously given Nembutal for general anesthesia. Each of the rabbits was then locally anesthetized with 0.5% Xylocaine at the proximal metaphysis of both tibiae, each of the bones was exposed, a small aperture, 16 mm in length and 3 mm in width, was formed in the bone therethrough, and additional small holes were further formed in the bone approximately at the lengthwise midportion of the aperture in communication therewith. The demineralized bone powder, sterilized with cooled ethylene oxide gas or immersed in alcohol-ether and then dried, was applied as wetted with physiological saline over the A-W glass ceramic plate, which was then inserted into the aperture of the tibia. The bone powder was filled into the remaining clearances through the holes, and the skin was then closed.

The grafted tibia portion was removed 1, 2, 3 or 4 weeks after the grafting and sliced to a thickness of 150 micrometers to prepare hard tissue specimens, which were Giemsa-stained and observed microscopically. A contact microradiograph was also prepared with soft rays. The results obtained revealed the following. One week after the grafting, numerous cartilage cells were found conglomerating between the particles of bone powder around the ceramic plate, and two weeks thereafter, cartilage cells were found present as mingled with ossified portions. Three weeks after the grafting, osteoinduction began on the borders of the grafting region of demineralized bone powder and spread through the entire grafting region. In this stage, particles of the bone powder applied were found remaining only sparsely with almost complete disappearance of cartilage cells. The ossification was found to have been completed four weeks later, and none of particles of the bone powder remained.

Two to 25 weeks after the grafting, the grafted tibia portion was removed and dynamically checked for the strength of joint beween the ceramic plate and the bone freshly formed therearound. For this procedure, the upper and lower 2-mm portions were removed from the inserted ceramic plate, and the bone was also cut off from the opposite sides of the plate to leave the bone attached only to the front and rear ends of the ceramic plate. The plate was then tested for separation by the method of Nakamura et al. (Nakamura, T. et al., J. Biomed. Mater. Res., 19, 685-698 (1985).).

**Table 1**

| Period after grafting (weeks) | Ceramic plate only | | Ceramic plate with calcified bone powder | |
|---|---|---|---|---|
| | n | Force required for separation | n | Force required for separation |
| 2 | 3^{a)} | 0.98 ± 0.67 kg | 6^{b)} | 2.58 ± 0.84 kg*** |
| 4 | 6 | 2.86 ± 1.11 kg | 7 | 7.21 ± 0.73 kg*** |
| 8 | 8 | 7.43 ± 1.03 kg | 8 | 9.00 ± 1.33 kg** |
| 12 | 7 | 8.19 ± 1.18 kg | 8 | 9.92 ± 1.11 kg*** |
| 25 | 8 | 7.14 ± 2.28 kg | 8 | 11.91 ± 2.76 kg* |
| Normal bone | 8 | 11.96±2.23 kg | | |

| | | | | |
|---|---|---|---|---|
| a) Average of 3 cases among 6 cases, not measurable in the other 3 cases due to failure to fix. | | | | |
| b) Average of 6 cases among 7 cases, not measurable in the remaining case due to failure to fix. Significant difference of the average | | | | |
| *: P < 0.01, | | | | |
| **: P < 0.005, | | | | |
| ***: P < 0.001 (t-test) N: Number of animals used. | | | | |

The results given in Table 1 show that the A-W glass ceramic plates with the demineralized powder applied thereto formed a joint of very high strength with the bone formed unlike the plates with no powder.

## Claims (Claims for the following Contracting State(s): AT, BE, CH, DE, FR, GB, IT, LI, LU, NL, SE)

1. A bone repairing material which comprises an AW-glass ceramic and an osteogenesis promoting substance selected from the group consisting of (1)a demineralized bone powder and (2)a substance containing bone bonding regulating factors, obtained by subjecting the demineralized bone powder to extraction with on aqueous solution of guanidine hydrochloric acid, subjecting the extract to gel filtration to obtain a fraction having osteogenetic activity and lyophilizing the fraction, and applied to and/or incorporated in the AW-glass ceramic.

2. The material of claim 1 wherein the osteogenesis promoting substance is applied to and/or incorporated in the AW-glass ceramic at the amount of 0.1 to 1.5 g/cm² based on the area of the portion thereof.

3. The material of claim 1 and 2 wherein the osteogenesis promoting substance is derived from the shaft of the long bone of a rabbit,bovine or neonatal calf.

4. The material according to anyone of claims 1-3 wherein the AW-glass ceramic has a porous surface suited to the application or incorporation of the osteogenesis promoting substance.

5. The material according to anyone of claims 1-4, wherein the osteogenesis promoting substance is & demineralized bone powder.

6. The material according to anyone of claims 1-4, wherein the osteogenesis promoting substance is a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction.

7. A process for preparing a bone repairing material comprising applying to and/or incorporating in an AW-glass ceramic an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction.

8. A use of an AW-glass and an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenesis activity and lyophilizing the fraction, for preparing a bone repairing material for inducing bone formation at a predetermined site in a mammal comprising implanting said AW-glass ceramic at the site, wherein said ceramic has said osteogenesis promoting substance applied to and/or incorporated in.

## Claims (Claims for the following Contracting State(s): ES, GR)

1. A process for preparing a bone repairing material comprising applying to and/or incorporating in an AW-glass ceramic an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction.

2. The process of claim 1 wherein the osteogenesis promoting substance is applied to and/or incorporated in the AW-glass ceramic at the amount of 0.1 to 1.5 g/cm² based on the area of the portion thereof.

3. The process of claim 1 and 2 wherein the osteogenesis promoting substance is derived from the shaft of the long bone of a rabbit, bovine or neonatal calf.

4. The process according to anyone of claims 1-3 wherein the AW-glass ceramic has a porous surface suited to the application or incorporation of the osteogenesis promoting substance.

5. The process according to anyone of claims 1-4, wherein the osteogenesis promoting substance is a demineralized bone powder.

6. The process according to anyone of claims 1-4, wherein the osteogenesis promoting substance is a substance containing bone bonding regulating factors, obtained by subjecting the demineralized bone powder to extraction with an aqueous solution of guanidine hydrochloric acid, subjecting the extract to gel filtration to obtain a fraction having osteogenetic activity and lyophilizing the fraction.

7. A use of an AW-glass and an osteogenesis promoting substance selected from the group consisting of (1)a demineralized bone powder and (2)a substance containing bone bonding regulating factors, obtained by subjecting the demineralized bone powder to extraction with an aqueous solution of guanidine hydrochloric acid, subjecting the extract to gel filtration to obtain a fraction having osteogenesis activity and lyophilizing the fraction, for preparing a bone repairing material for inducing bone formation at a predetermined site in a mammal comprising implanting said AW-glass ceramic at the site, wherein said ceramic has said osteogenesis promoting substance applied to and/or incorporated in.

## Patentansprüche (Patentansprüche für folgende(n) Vertragsstaat(en): AT, BE, CH, DE, FR, GB, IT, LI, LU, NL, SE)

1. Knochenreparaturmaterial, umfassend eine AW-Glaskeramik und eine die Knochenbildung fördernde Substanz, die aus der Gruppe ausgewählt ist, bestehend aus (1) einem entmineralisierten Knochenpulver und (2) einer Substanz, die Knochenverbindung regulierende Faktoren enthält, erhalten durch Extrahieren des entmineralisierten Knochenpulvers mit einer wäßrigen Lösung von Guanidin-Hydrochlorld, Durchführen einer Gel-Filtration mit dem Extrakt, um eine Fraktion mit knochenbildender Aktivität zu erhalten, sowie Lyophilisieren der Fraktion, und die auf die AW-Glaskeramik aufgetragen und/oder eingebaut wird.

2. Material gemäß Anspruch 1, wobei die Knochenbildung fördernde Substanz in einer Menge von 0,1 bis 1,5 g/cm², auf die AW-Glaskeramik aufgetragen und/oder eingebaut wird, bezogen auf die Fläche des betreffenden Teils derselben.

3. Material gemäß Anspruch 1 und 2, wobei die Knochenbildung fördernde Substanz aus dem Schaft des langen Knochens eines Kaninchens, Rindes oder neugeborenen Kalbes stammt.

4. Material gemäß einem der Ansprüche 1-3, worin die AW-Glaskeramik eine poröse Oberfläche hat, die sich für die Auftragung oder den Einbau der die Knochenbildung fördernden Substanz eignet.

5. Material gemäß einem der Ansprüche 1-4, worin die Knochenbildung fördernde Substanz ein entmineralisiertes Knochenpulver ist.

6. Material gemäß einem der Ansprüche 1-4, worin die Knochenbildung fördernde Substanz eine Substanz ist, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion.

7. Verfahren zur Herstellung eines Knochenreparaturmaterials, umfassend das Auftragen und/oder den Einbau einer die Knochenbildung fördernden Substanz, die aus der Gruppe ausgewählt ist, bestehend aus (1) einem entmineralisierten Knochenpulver und (2) einer Substanz, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion, auf bzw. in eine AW-Glaskeramik.

8. Verwendung einer AW-Glaskeramik und einer die Knochenbildung fördernden Substanz, die aus der Gruppe ausgewählt ist, bestehend aus (1) einem entmineralisierten Knochenpulver und (2) einer Substanz, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion, zur Herstellung eines Knochenreparaturmaterials zum Induzieren der Knochenbildung an einer vorherbestimmten Stelle in einem Säuger, umfassend das Implantieren der AW-Glaskeramik in die Stelle, wobei die Keramik die Knochenbildung fördernde Substanz aufgetragen und/oder eingebaut hat.

## Patentansprüche (Patentansprüche für folgende(n) Vertragsstaat(en): ES, GR)

1. Verfahren zur Herstellung eines Knochenreparaturmaterials, umfassend das Auftragen und/oder den Einbau einer die Knochenbildung fördernden Substanz, die aus der Gruppe ausgewählt ist, bestehend aus (1) einem entmineralisierten Knochenpulver und (2) einer Substanz, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion, auf bzw. in eine AW-Glaskeramik.

2. Verfahren gemäß Anspruch 1, wobei die Knochenbildung fördernde Substanz in einer Menge von 0,1 bis 1,5 g/cm², auf die AW-Glaskeramik aufgetragen und/oder eingebaut wird, bezogen auf die Fläche des betreffenden Teils derselben.

3. Verfahren gemäß Anspruch 1 und 2, wobei die Knochenbildung fördernde Substanz aus dem Schaft des langen Knochens eines Kaninchens, Rindes oder neugeborenen Kalbes stammt.

4. Verfahren gemäß einem der Ansprüche 1-3, worin die AW-Glaskeramik eine poröse Oberfläche hat, die sich für die Auftragung oder den Einbau der die Knochenbildung fördernden Substanz eignet.

5. Verfahren gemäß einem der Ansprüche 1-4, worin die Knochenbildung fördernde Substanz ein entmineralisiertes Knochenpulver ist.

6. Verfahren gemäß einem der Ansprüche 1-4, worin die Knochenbildung fördernde Substanz eine Substanz ist, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion.

7. Verwendung einer AW-Glaskeramik und einer die Knochenbildung fördernden Substanz, die aus der Gruppe ausgewählt ist, bestehend aus (1) einem entmineralisierten Knochenpulver und (2) einer Substanz, die die Knochenverbindung regulierende Faktoren enthält, erhalten durch Extrahieren des entmineralisierten Knochenpulvers mit einer wäßrigen Lösung von Guanidin-Hydrochlorid, Durchführen einer Gel-Filtration mit dem Extrakt, um eine Fraktion mit knochenbildender Aktivität zu erhalten, sowie Lyophilisieren der Fraktion, zur Herstellung eines Knochenreparaturmaterials zum Induzieren der Knochenbildung an einer vorherbestimmten Stelle in einem Säuger, umfassend das Implantieren der AW-Glaskeramik in die Stelle, wobei die Keramik die Knochenbildung fördernde Substanz aufgetragen und/oder eingebaut hat.

## Revendications (Revendications pour l'(les) Etat(s) contractant(s) suivant(s): AT, BE, CH, DE, FR, GB, IT, LI, LU, NL, SE)

1. Matériau de réparation des os qui contient une cérarmique de verte AW et une substance favorisant l'ostéogénèse choisie dans le groupe formé par (1) une poudre d'os déminéralisée et (2) une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction, et appliquée sur la céramique de verre AW ou incorporée à celle-ci.

2. Matériau selon la revendication 1, dans lequel la substance favorisant l'ostéogénèse est appliquée sur la céramique de verre AW, ou lui est incorporée, à raison de 0,1 à 1,5 g/cm² rapportés à l'aire de la partie de celle-ci.

3. Matériau selon la revendication 1 et 2 dans lequel la substance favorisant l'ostéogénèse est dérivée du corps d'un os long de lapin, de bovin ou de veau nouveau-né.

4. Matériau selon l'une quelconque des revendications 1 à 3, dans lequel la céramique de verre AW a une surface poreuse appropriée à l'application ou l'incorporation de la substance favorisant l'ostéogénèse.

5. Matériau selon l'une quelconque des revendications 1 à 4, dans lequel la substance favorisant l'ostéogénèse est une poudre d'os déminéralisée.

6. Matériau selon l'une quelconque des revendications 1 à 4, dans lequel la substance favorisant l'ostéogénèse est une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction.

7. Procédé de préparation d'un matériau de réparation des os qui comprend l'application sur une céramique de verte AW, ou l'incorporation à celle-ci, d'une substance favorisant l'ostéogénèse choisie dans le groupe formé par (1) une poudre d'os déminéralisée et (2) une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction.

8. Utilisation d'un verte AW et d'une substance favorisant l'ostéogénèse choisie dans le groupe formé par (1) une poudre d'os déminéralisée et (2) une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction, pour préparer un matériau de réparation des os destiné à induire la formation d'os en un site prédéterminé d'un mammifère, comprenant l'implantation de ladite céramique de verte AW au niveau du site, sachant que ladite substance favorisant l'ostéogénèse est appliquée sur ladite céramique ou incorporée dans celle-ci.

## Revendications (Revendications pour l'(les) Etat(s) contractant(s) suivant(s): ES, GR)

1. Procédé de préparation d'un matériau de réparation des os qui comprend l'application sur une céramique de verre AW, ou l'incorporation à celle-ci, d'une substance favorisant l'ostéogénèse choisie dans le groupe formé par (1) une poudre d'os déminéralisée et (2) une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction.

2. Procédé selon la revendication 1, dans lequel la substance favorisant l'ostéogénèse est appliquée sur la céramique de verre AW, ou lui est incorporée, à raison de 0,1 à 1,5 g/cm² rapportés à l'aire de la partie de celle-ci.

3. Procédé selon la revendication 1 et 2 dans lequel la substance favorisant l'ostéogénèse est dérivée du corps d'un os long de lapin, de bovin ou de veau nouveau-né.

4. Procédé selon l'une quelconque des revendications 1 à 3, dans lequel la céramique de verre AW a une surface poreuse appropriée à l'application ou l'incorporation de la substance favorisant l'ostéogénèse.

5. Procédé selon l'une quelconque des revendications 1 à 4, dans lequel la substance favorisant l'ostéogénèse est une poudre d'os déminéralisée.

6. Procédé selon l'une quelconque des revendications 1 à 4, dans lequel la substance favorisant l'ostéogénèse est une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction.

7. Utilisation d'un verre AW et d'une substance favorisant l'ostéogénèse choisie dans le groupe formé par (1) une poudre d'os déminéralisée et (2) une substance contenant des facteurs de régulation de la fixation osseuse, obtenue par soumission de la poudre d'os déminéralisée à une extraction avec une solution aqueuse de chlorhydrate de guanidine, soumission de l'extrait à une filtration sur gel pour obtenir une fraction ayant une activité ostéogénique et lyophilisation de la fraction, pour préparer un matériau de réparation des os destiné à induire la formation d'os en un site prédéterminé d'un mammifère, comprenant l'implantation de ladite céramique de verre AW au niveau du site, sachant que ladite substance favorisant l'ostéogénèse est appliquée sur ladite céramique ou incorporée dans celle-ci.
